# EUROPÄISCHE PATENTANMELDUNG

(11) **EP 1 226 808 A2**
(43) Veröffentlichungstag der Anmeldung: **31.07.2002**
(21) Anmeldenummer: 01129666.2
(22) Anmeldetag: 13.12.2001
(51) Int. Cl.: A61K 6/10

(54) **Verwendung von Mischungen als Abform- oder Dubliermassen im Dentalbereich**

(30) Priorität: 29.01.2001 DE 10104079
(71) Anmelder: Heraeus Kulzer GmbH & Co.KG, 63450 Hanau (DE)
(72) Erfinder: Schaub, Matthias, Dr., 40593 Düsseldorf (DE); Urbas, Holger, 47829 Krefeld (DE); Freckmann, Michael, 50769 Köln (DE)
(74) Vertreter: Kühn, Hans-Christian

(57) **Zusammenfassung**

Verwendung von Mischungen, enthaltend
A) alkoxysilylfunktionelle Polyether mit linearer oder verzweigter Hauptkette mit einem mittleren Molekulargewicht (Mₙ) von 800 - 20000, mit
   einem Gehalt an Polyethergruppen von 20 - 95 %,
   einem Gehalt an Alkoxysilylgruppen -SiR¹R²R³ von 0,2 - 25 %,
   wobei R¹, R² und R³ unabhängig voneinander H, alkyl oder alkoxy bedeuten,
   einem Gehalt an Urethangruppen von 0 - 10 % oder einem Gehalt an Harnstoffgruppen von 0 - 10 %,
   und
B) eine Mischung, enthaltend Wasser und organische und/oder anorganische Säuren in Gewichtsverhältnissen von 1 : 0,01 bis 1 : 40,
   als Abform- oder Dubliermassen im Dentalbereich.

## Beschreibung

Die Erfindung betrifft die Verwendung von Mischungen als Abform- oder Dubliermassen im Dentalbereich.

Abformmassen, die im Dentalbereich Anwendung finden, sind an sich bekannt (siehe R. G. Craig, Restaurative Dental Materials, The C. V. Moosbe-Comp. St. Louis, Toronto, London, 1980, S 1979 ff). An derartige Materialien werden insgesamt sehr hohe Anforderungen gestellt:
1. Angenehmer Geruch, Geschmack und ästhetisches Aussehen.
2. Die Massen dürfen keine toxischen oder irritierenden Bestandteile enthalten.
3. Die Massen müssen eine mehrmonatige Lagerstabilität aufweisen.
4. Die Massen müssen wirtschaftlich herstellbar sein und eine präzise Abformung ergeben.
5. Die Massen müssen leicht zu handhaben sein.
6. Die Härtungscharakteristik muss den klinischen Erfordernissen entsprechen.
7. Die ausgehärteten Massen müssen elastisch sein und dürfen sich nicht unter Zugbeanspruchung bleibend verformen.
8. Die ausgehärteten Massen müssen eine ausreichende Druckfestigkeit besitzen und dürfen nicht brechen.
9. Die ausgehärteten Massen müssen bei Raumtemperatur und normaler Luftfeuchtigkeit solange dimensionsstabil sein, dass in angemessener Zeit exakte Gipsabdrücke hergestellt werden können.
10. Die ausgehärteten Massen dürfen keine Gipsschädigung hervorrufen und müssen mit anderen Abformmassen kompatibel sein.

Abformmassen auf Basis alkoxysilylfunktionalisierter Polyether werden in EP 0 269 819 B1 beschrieben.

Dort wird die Verwendung von Mischungen offenbart, enthaltend
A) ein Ether-, Urethan-, Harnstoffgruppen und Alkoxysilyendgruppen enthaltendes Polyadditionsprodukt mit einer überwiegend linearen Molekülstruktur, ausschließlich aliphatisch oder cycloaliphatisch gebundenen Ether-, Urethan- und Harnstoffsegmenten und einem mittleren Molekulargewicht Mn von 800 - 2000, gekennzeichnet durch
   a) einen Gehalt von Polyethergruppen von 25 - 90 Gewichts-%,
   b) einen Gehalt von Urethangruppen (-NH-CO-O-) von 0,5 - 10 Gewichts-%,
   c) einen Gehalt von Harnstoffgruppen (-NH-CO-NH-) von 0,5 -10 Gewichts-% und
   d) endständige Gruppen der Formel -NR-(CH₂)ₙ-SiR₁R₂R₃,
   worin n die Zahlen 1 bis 6 darstellt,
   R Wasserstoff oder -(CH₂)ₙ-SiR₁R₂R₃ bedeutet,
   R₁R₂R₃ unabhängig voneinander C1 - C4-Alkoxy bedeuten, wobei der Gehalt der endständigen Alkoxysilylgruppen -SiR₁R₂R₃ 1 - 25 Gewichts-% beträgt und
B) eine Abmischung, enthaltend Wasser und organische und/oder anorganische Säuren in Gewichtsmengenverhältnissen von 1 : 0,01 bis 1 : 40, als Abform- oder Dubliermassen im Dentalbereich.

Diese Systeme erfüllen jedoch nicht alle der oben genannten Anforderungen im wünschenswerten Ausmaß.

Insbesondere sind diese Abformmassen durch mehrstufige aufwendige Verfahren zu synthetisieren, wobei darüber hinaus die Produkte hochviskos sind, da Diisocyanate mit Dihydroxypolyethern umgesetzt werden, wobei es zwangsläufig zu einem Anstieg des mittleren Molekulargewichts (Mₙ) und damit auch zu einem Anstieg der Viskosität kommt. Damit sind die Möglichkeiten der Formulierung pastöser Massen mit den aus dem Stand der Technik bekannten Polyadditionsprodukten stark eingeschränkt, da entweder hohe Verdünnergehalte oder geringe Füllstoffgehalte eingestellt werden müssen, um verarbeitbare - also nicht zu hochviskose - Massen zu erhalten.

Schließlich zeigen die Abformmassen auf Basis der in EP 0 269 819 B1 beschriebenen Polyadditionsprodukte bei Abmischung mit Wasser und Säure eine ungünstige Aushärtungskinetik, die durch eine kurze Verarbeitungszeit und gleichzeitig durch eine lange Abbindezeit gekennzeichnet ist.

In DE 44 39 769 werden Kunststoffe offenbart mit mindestens einem Silan-, Ether- und Urethangruppen enthaltenden Polyadditionsprodukt mit einer überwiegend linearen Molekülstruktur mit aliphatisch oder cycloaliphatisch gebundenen Ether- oder Urethansegmenten und einem Zahlenmittel der Molmasse im Bereich von 800 bis 20000, wobei das Polyadditionsprodukt die folgenden Merkmale aufweist:
a) einen Gehalt an Polyethergruppen von 20 bis 90, insbesondere 50 bis 80 Gew.-Teilen, auf 100 Gew.-Teile Polyadditionsprodukt;
b) einen Gehalt an Urethangruppen der Formel I

   - HN - CO - O (I)

   von 0,5 bis 10, insbesondere von 1 bis8 Gew.-Teilen auf 100 Gew.Teile Polyadditionsprodukt;
c) sowie einen Gehalt an endständig angeordneten Alkoxysilylgruppen der Formel II

   - NR - (CH₂)ₘ -SiR¹R²R³ (II)

   in der
   m eine Zahl im Bereich von 1 bis 6, insbesondere 3,
   R Wasserstoff oder eine Gruppe der Formel (III)

   - (CH₂)ₘ - SiR¹R²R³ (III)

   mit den hier angegebenen Bedeutungen für m, R¹, R² und R³, und mindestens eine der Gruppen R¹, R² und R³ eine Gruppe der Formel IV

   - (O - CₚH₂ₚ)_{q} - O - R⁴ (IV)

   in der
   p eine Zahl im Bereich von 2 bis 4, insbesondere 3, und
   q eine Zahl im Bereich von 1 bis 100, insbesondere von 2 bis 4 sowie
   R⁴ eine Alkyl-, Aralkyl-, Vinyl-, Vinylcarbonyl, alpha-Methylvinylcarbonyl- oder beta-Methylvinylcarbonylgruppe ist, bedeuten,
wobei die restlichen Gruppen R¹, R² und R³ Methyl, Ethyl oder C₁- bis C₄-Alkoxy bedeuten, soweit sie nicht Gruppen der obigen Definitionen sind,
und die Kunststoffe weiterhin mindestens einen Katalysator für die Kondensation der Silangruppen enthalten.

Nachteil dieser Systeme ist ebenfalls ein aufwendiges mehrstufiges Herstellverfahren, das einen Umesterungsschritt, kommerziell erhältlicher Silane mit Verbindungen der Struktur H-(O-CₚH₂ₚ)_{q}-O-R⁴ erfordert. Ein weiterer Nachteil dieser Systeme ist das hohe toxische Potential der im Laufe der Abbindereaktion freigesetzten Gruppen der allgemeinen Formel H-(O-CₚH₂ₚ)_{q}-O-R⁴.

Problem der vorliegenden Erfindung ist es, die Nachteile der bekannten Abformmassen auf Basis alkoxysilylfunktioneller Polyether zu vermeiden, also insbesondere Abformmassen auf Basis kommerziell erhältlicher oder einfach (vorzugsweise in einem Reaktionsschritt) zu synthetisierender, niedrigviskoser (Viskosität < 50 Pas) alkoxysilyfunktioneller Polyether zur Verfügung zu stellen, die sich durch eine günstige Abbindekinetik (d. h. Verarbeitungszeiten im Bereich von 2 bis 3 Minuten bei Aushärtezeiten von < 4 Minuten) also durch ein sogenanntes Snap-Set-Verhalten (langsame Induktionszeit von bis zu mehreren Minuten und anschließend sehr schnelles Abbinden) auszeichnen.

Dieses Problem wird erfindungsgemäß durch eine Verwendung nach Anspruch 1 gelöst.

Hierbei handelt es sich um die Verwendung von Mischungen, enthaltend.
A) alkoxysilylfunktionelle Polyether mit linearer oder verzweigter Hauptkette mit einem mittleren Molekulargewicht (Mₙ) von 800 - 20000, mit
   einem Gehalt an Polyethergruppen von 20 - 95 %,
   einem Gehalt an Alkoxysilylgruppen -SiR¹R²R³ von 0,2 - 25 %,
   wobei R¹, R² und R³ unabhängig voneinander H, alkyl oder alkoxy bedeuten,
   einem Gehalt an Urethangruppen von 0 - 10 % oder einem Gehalt an Harnstoffgruppen von 0-10%,
   und
B) eine Mischung, enthaltend Wasser und organische und/oder anorganische Säuren in Gewichtsverhältnissen von 1 : 0,01 bis 1 : 40,
   als Abform- oder Dubliermassen im Dentalbereich.

In weiteren Ausführungsformen der Erfindung werden
2. Mischungen verwendet, deren Komponente A) eine verzeigte Hauptkette aufweist;
3. Mischungen verwendet, deren Komponente A) frei von Urethangruppen ist:
4. Mischungen verwendet, deren Komponente A) ein mittleres Molekulargewicht von 1500 bis 15000 aufweist;
5. Mischungen verwendet, deren Komponente A) einen Gehalt an Alkoxysilylgruppen - SiR¹R²R³ von 2-15 % aufweist.

Erfindungsgemäß eingesetzte alkoxysilylfunktionelle Polyether sind zum Teil kommerziell erhältlich, z.B. MS Polymer der Kanaka Corporation. Hierbei handelt es sich um Polypropylenoxidderivate, die mit Methyldimethoxysilylresten funktionalisiert sind (z. B. MS Polymer S303H).

Weiterhin können erfindungsgemäß eingesetzte Polyether hergestellt werden, in dem lineare oder verzweigte Polyether-Polyole bzw. lineare oder verzweigte aminoendständige Polyether mit geeignet funktionalisierten Alkoxysilanen und gegebenenfalls Polyisocyanaten bei Temperaturen von 20 - 150°C umgesetzt werden. Hierbei kann die Verwendung eines Katalysators erforderlich sein.

Geeignet zur Herstellung der erfindungsgemäßen alkoxysilylfunktionellen Polyether sind z. B. Polyether-Polyole, die aus der Polyurethan-Herstellung bekannt sind (z. B. Ullmann's Encyclopedia of Industrial Chemistry, Vol. A 21, S. 665 - 717, VCH Publishers Inc. 1992; oder US 5,672,652,). Hierbei handelt es sich um Verbindungen, die durch Polymerisation von Epoxiden wie Ethylenoxid, Propylenoxid, Butylenoxid, Tetrahydrofuran, Styroloxid oder Epichlorhydrin mit sich selbst, z. B. in Gegenwart von BF3 oder durch Anlagerung dieser Epoxide gegebenenfalls im Gemisch oder nacheinander an Starterkomponenten mit reaktionsfähigen Wasserstoffatomen wie Alkohole, Glykole, Glycerin, Trimethylolpropan, Pentaerythrit, Zucker, Ethylendiamin, Diethylentriamin etc., hergestellt werden. Vielfach sind solche Polyether bevorzugt, die überwiegend (bis zu 90 % bezogen auf alle vorhandenen OH-Gruppen im Polyether) primäre OH-Gruppen aufweisen. Besonders bewährt haben sich Polyether, die durch die sogenannte DMC-Katalyse, z. B. mit Zinkhexacyanokobaltat (US-A 3, 278, 457) hergestellt werden. In einer bevorzugten Ausführungsform weisen die eingesetzten Polyether-Polyole ein Molekulargewicht (Mn) von etwa 1000 bis etwa 15000 auf und zeichnen sich durch eine OH-Funktionalität von etwa 1,5 bis 4 aus.

Geeignet zur Herstellung der erfindungsgemäßen alkoxysilylfuntkionellen Polyether sind weiterhin sogenannte amino-terminierte Polyether, die ebenfalls aus dem Bereich der Polyurethan-Herstellung bekannt sind. Amino-terminierte Polyether werden ausgehend von Polyether-Polyolen durch Austausch der OH-Gruppen gegen Ammoniak oder primäre Amine erhalten (z. B. US 3 847 992). Zur Herstellung von amino-terminierten Polyethern können im Prinzip die oben aufgeführten Polyether-Polyole als Ausgangsverbindungen eingesetzt werden. In einer bevorzugten Ausführungsform weisen die eingesetzten amino-terminierte Polyether ein Molekulargewicht (Mn) von etwa 500 bis etwa 15000 und eine Amino-Funktionalität von etwa 2 bis 4 auf.

Geeignete funktionalisierte Alkoxysilane zur Herstellung der erfindungsgemäßen alkoxysilylfunktionaliserten Polyether zeichnen sich durch folgende Struktur aus:

X-(CH2)ₙ-SiR¹ R²R³,

dabei steht X für eine mit einer Hydroxy- oder Aminogruppe reaktionsfähige Gruppe, n steht für eine Zahl von 1 bis 8, und R¹, R² und R³ bedeuten unabhängig voneinander H, alkyl, oder alkoxy. Bevorzugte Alkoxysilane sind 3-lsocyanatopropyl-triethoxysilan und 3-lsocyanatopropyltrimethoxysilan.

Geeignete Polyisocyanate sind die aus der Polyurethan-Chemie bekannten aliphatischen Systeme wie Ethylendiisocyanat, Tetramethylendiisocyanat, Hexamethylendiisocyanat, Dodecamethylendiisocyanat, Cyclobutan-1,3-diisocyanat, Cyclohexan-1,3-diisocyanat, Cyclohexan-1,4-diisocyanat, 1-Isocyanato-3,3,5-trimethyl-5-isocyanatomethyl-cyclohexan (Isophorondiisocyanat), N-lsocyanatohexylaminocarbonyl-N,N'-bis-(isocyanatohexyl)harnstoff, 1,1-Methylen-bis-(4-isocyanatocyclohexan), 4,4'-diisocyanatodicyclohexylmethan, 2,4,6-Trioxo-1,3,5-tris(6-isocyanatohexyl)hexahydro-1,3,5-triazin oder 2,4,6-Trioxo-1 ,3,5-tris(5-isocyanato-1,3,3.trimethylcyclohexylmethyl)hexahydro-1,3,5-triazin.

Vorzugsweise werden beim erfindungsgemäßen Verfahren cycloaliphatische bzw. gemischt aliphatisch-cycloaliphatische Polyisocyanate eingesetzt. Besonders bevorzugt ist 1-lsocyanato-3,3,5-trimethyl-5-isocyanatomethyl-cyclohexan (Isophorondiisocyanat).

Geeignete Katalysatoren zur Herstellung der erfindungsgemäß eingesetzten alkoxysilylfunktionalisierten Polyether sind ebenfalls aus der Polyurethan-Chemie bekannt (z.B. Ullmann's Encyclopedia of Industrial Chemistry, Vol. A 21, S. 665 - 717, VCH Publishers Inc. 1992). Dabei handelt es sich z.B. um Lewis-Basen, wie 1,4-Diazabicyclo[2.2.2]octan (DABCO) oder 1,8-diazabicyclo[5.4.0]undec-7-en (DBU) oder Lewis-Säuren wie Dibutylzinndilaurat (DBTL) oder Zinn-dioctoat. Bevorzugt ist die katalysatorfreie Herstellung der erfindungsgemäß eingesetzten alkoxysilylfunktionalisierten Polyether.

Durch die Abmischung von alkoxysilylfunktionalisierten Polyethern (Komponente A) mit Wasser und organischen und/oder anorganischen Säuren in Gewichtsverhältnissen von 1 : 0,1 bis 1 : 40 (Komponente B) werden elastomere Produkte erhalten, die in hervorragender Weise als Abform- oder Dubliermassen im Dentalbereich verwendet werden können.

Sowohl die Komponente A als auch die Komponente B können weitere übliche Hilfs- oder Zuschlagsstoffe zur Formulierung von pastösen Produkten enthalten. Unter Hilfs- und/oder Zuschlagsstoffen versteht man beispielsweise Verdünner, wie aromatische oder aliphatische Kohlenwasserstoffe, Alkohole, Ether, Polyether, Ester, Polyester, Füllstoffe, z.B. Quarzmehle, Cristobalithmehle, Calciumsulfat, Diatomeenerde, Silikate, gefälltes oder pyrogen hergestelltes Siliciumdioxid mit unbeladener oder beladener Oberfläche. Additive Farbstoffe; Geruchs- und Geschmacksstoffe, Emulgatoren, Stabilisatoren,

Bevorzugt werden die Komponenten A und B in Tuben, Schlauchbeuteln oder Doppelkartuschen zur Verwendung dargeboten.

Die Herstellung von elastomeren Abformungen auf Basis der erfindungsgemäßen Abformmassen kann in der dem Fachmann bekannten Weise erfolgen, wobei die Abmischung der Komponenten A und B per Hand oder mit Auto-Mixsystemen erfolgen kann. Für den Anwender sind die Parameter Verarbeitungszeit und Abbindezeit sehr wichtige Kenngrößen eines Abformmaterials. Erwünscht ist, dass bei einer praktikablen Verarbeitungszeit, die meist im Bereich weniger Minuten liegt eine Abbindezeit eingestellt werden kann, die nur wenig über der Verarbeitungszeit liegt. Die Abbindecharakteristik elastomerer Abformmassen lässt sich z. B. mittels rheometrischer Methoden quantifizieren Durch Messungen mittels Oszillationsrheometrie lassen sich die Beträge von Speichermodul G' (als Maß für den elastischen Anteil eines Abformmaterials) und von Verlustmodul G" (als Maß für den viskosen Anteil eines Abformmaterials) ermitteln. Weiterhin gilt tanδ = (G"/G'). Durch Beobachtung des Aushärtungsverlaufs in Abhängigkeit von der Zeit nach Mischbeginn mittels Oszillationsrheometrie lassen sich die Zeiten ermitteln, bei denen gilt G' = G", oder δ = 45° (t₁) und δ = 10° (t₂); dabei kann t1 als Verarbeitungszeit und t2 als Aushärtezeit des Abformmaterials interpretiert werden. Das Verhältnis t₂/t₁ kann als Größe zur Charakterisierung der Abbindekinetik betrachtet werden. Je näher dieser Quotient an 1, desto günstiger ist das Abbindeverhalten in Richtung "Snap-Set" eingestellt. Während für Abformmassen auf Basis alkoxysilylfunktioneller Polyether entsprechend dem Stand der Technik ein Verhältnis t₂/t₁ im Bereich von 1,7 - 2,0 gefunden wird, kann bei den erfindungsgemäßen Systemen ein Verhältnis t₂/t₁ von 1,2 - 1,3 erreicht werden.

Es ist äußerst überraschend, dass die erfindungsgemäßen Abformmassen sich gegenüber dem Stand der Technik durch eine wesentlich günstigere Abbindekinetik auszeichnen.

### Beispiele

### Beispiel 1:

### Synthese von alkoxysilylfunkktionalisierten Polyethern aus Polyetherpolyolen Allgemeine Vorschrift:

300 g eines Polyether-Polyols werden für 1 Stunde bei 10 mbar und 100 °C entwässert. Danach werden pro Mol Hydroxygruppe 1 Mol Isocyanatopropyltriethoxysilan zugefügt und anschließend 1 Tropfen Dibutylzinndilaurat zugesetzt. Der Ansatz wird bei 100°C solange gerührt, bis keine Isocyanatgruppen mehr nachweisbar sind. (s. Tabelle 1, A1 - A5).

### Beispiel 2:

### Synthese von alkoxysilylfunktionalisierten Polyethern aus aminoendständigen Polyethern

### Allgemeine Vorschrift:

300 g eines aminoendständigen Polyethers werden für eine Stunde bei 10 mbar und 100°C entwässert. Nach Abkühlen auf 60 °C wird ein Gemisch aus Isocyanatopropyltrialkoxysilan, Isophorondiisocyanat und Cyclohexylisocyanat über einen Zeitraum von 30 Minuten zugetropft, so dass das molare Verhältnis von Aminogruppen zu Isocyanatgruppen 1 : 1 beträgt. Der Ansatz wird bei 60 °C noch solange nachgerührt, bis keine Isocyanatgruppen mehr nachweisbar sind (s. Tabelle 1, A7 - A11).

### Vergleichsbeispiel:

Die Synthese eines linearen Poly(ether-urethan-harnstoff)-Polyadditionsproduktes gemäß EP 0 269 819 B1, Beispiel 3, erforderte zwei Reaktionsschritte und führt zu einem sehr hochviskosen Endprodukt (s. Tabelle 1, V1).

### Beispiel 3:

### Formulierung der Katalysator-Komponente B

In einem Mischwerk werden

| | |
|---|---|
| 32,2 % | Poly(propylenoxid)-diol (MW 2000) |
| 2,8% | Paraffin |
| 0,3% | Emulgator |
| 59,2% | Quarzmehl |
| 3,2% | pyrogene Kieselsäure |
| 2,3% | einer 16%igen wässrigen Lösung von p-Toluolsulfonsäurehydrat |

in der angegebenen Reihenfolge zu einer homogenen pastösen Masse gemischt. Die Mischzeit beträgt 30 Minuten bei 50 U/min.

### Beispiel 4:

Die in den Beispielen 1 und 2 beschriebenen alkoxysilyl-endständigen Polyether und kommerziell erhältliche alkoxysilyl-endständigen Polyether werden als Komponente A im Gewichts-Verhältnis 1 : 1 mit der Katalysator-Komponente B intensiv vermischt. Nach einigen Minuten erhält man ein zu einem elastischen Material vernetztes Produkt.

### Beispiel 5:

### Formulierung einer dentalen Abformmassen

In einem Mischwerk werden

| | |
|---|---|
| 19,1 % | A10 |
| 19,1 % | Verdünner |
| 54,5 % | Füllstoff (Quarzmehl) |
| 4,0 % | Paraffin |
| 3,8 % | Polyethylen-Faser |

zu einer pastösen Masse homogenisiert (Komponente A).

Die Komponenten A und B werden im Gewichts-Verhältnis 8:1 für 30s intensiv vermischt.

Die physikalische Charakterisierung der Abformmasse nach ISO 4823 liefert folgende Ergebnisse:

| | |
|---|---|
| Viskosität | 153,6 |
| Verarbeitungszeit [min] | 1,8 |
| Aushärtezeit [min] | 2,4 |
| Aushärtezeit/Verarbeitungszeit | 1,3 |
| Rückstellung nach Verformung [%] | 97,7 |
| Dimensionsänderung [%] | 0,45 |
| Shore A-Härte (1 Stunde) | 52 |

### Vergleichsbeispiel:

In einem Mischwerk werden

| | |
|---|---|
| 19,1 % | V 1 |
| 19,1 % | Verdünner |
| 54,5 % | Füllstoff (Quarzmehl) |
| 4,0 % | Paraffin |
| 3,8 % | Polyethylen-Faser |

zu einer pastösen Masse homogenisiert (Komponente A).

Die Komponenten A und B werden im Gewichts-Verhältnis 5 : 1 für 30 Sekunden intensiv vermischt. Die Untersuchungen zur Abbindekinetik und die physikalische Charakterisierung der Abformmasse nach ISO 4823 liefern folgende Ergebnisse:

| | |
|---|---|
| Viskosität (23°C, 3s 1) [Pas] | 275 |
| Verarbeitungszeit [min] | 2,2 |
| Aushärtezeit [min] | 3,8 |
| Aushärtezeit/Verarbeitungszeit | 1, 7 |
| Rückstellung nach Verformung [%] | 98 |
| Dimensionsänderung [%] | 0,36 |
| Shore A-Härte (1 h) | 51 |

## Patentansprüche

1. Verwendung von Mischungen, enthaltend
A) alkoxysilylfunktionelle Polyether mit linearer oder verzweigter Hauptkette mit einem mittleren Molekulargewicht (Mₙ) von 800 - 20000, mit
einem Gehalt an Polyethergruppen von 20 - 95 %,
einem Gehalt an Alkoxysilylgruppen -SiR¹R²R³ von 0,2 - 25 %,
wobei R¹, R² und R³ unabhängig voneinander H, alkyl oder alkoxy bedeuten,
einem Gehalt an Urethangruppen von 0 - 10 % oder einem Gehalt an Harnstoffgruppen von 0 - 10 %,
und
B) eine Mischung, enthaltend Wasser und organische und/oder anorganische Säuren in
Gewichtsverhältnissen von 1 : 0,01 bis 1 : 40,
als Abform- oder Dubliermassen im Dentalbereich.

2. Verwendung von Mischungen nach Anspruch 1, **dadurch gekennzeichnet, dass** die Komponente A) eine verzweigte Hauptkette aufweist.

3. Verwendung von Mischungen nach Anspruch 1 oder 2, **dadurch gekennzeichnet, dass** die Komponente A) frei von Urethangruppen ist.

4. Verwendung von Mischungen nach einem der Ansprüche 1 bis 3, **dadurch gekennzeichnet, dass** die Komponente A) ein mittleres Molekulargewicht (Mn) von 1500- 15000 aufweist.

5. Verwendung von Mischungen nach einem der Ansprüche 1 bis 4, **dadurch gekennzeichnet, dass** die Komponente A) einen Gehalt an Alkoxysilylgruppen -SiR¹R²R³ von 2 bis 15 % aufweist.

6. Verwendung von Mischungen nach einem der Ansprüche 1 bis 5, **dadurch gekennzeichnet, dass** die Darbietung der Komponenten A) und B) in Tuben, Schlauchbeuteln oder Doppelkartuschen erfolgt.
